(19) Europäisches Patentamt
European Patent Office
Office européen des brevets

(11) EP 0 648 102 B1

(12) **FASCICULE DE BREVET EUROPEEN**

(45) Date de publication et mention de la délivrance du brevet:
**20.03.1996 Bulletin 1996/12**

(21) Numéro de dépôt: **93914783.1**

(22) Date de dépôt: **30.06.1993**

(51) Int. Cl.$^6$: **A61K 7/00**

(86) Numéro de dépôt international: **PCT/FR93/00658**

(87) Numéro de publication internationale:
**WO 94/01073 (20.01.1994 Gazette 1994/03)**

(54) **COMPOSITION COSMETIQUE SOUS FORME D'EMULSION TRIPLE EAU/HUILE DE SILICONE/EAU GELIFIEE**

KOSMETISCHE ZUSAMMENSETZUNG IN FORM EINER DREIFACH- EMULSION VOM TYP WASSER/SILIKONÖL/GELIERTES WASSER

COSMETIC COMPOSITION IN THE FORM OF A GELLED SILICONE/WATER TRIPLE WATER/OIL EMULSION

(84) Etats contractants désignés:
**CH DE ES FR GB IT LI**

(30) Priorité: **09.07.1992 FR 9208532**

(43) Date de publication de la demande:
**19.04.1995 Bulletin 1995/16**

(73) Titulaire: **L'OREAL**
**F-75008 Paris (FR)**

(72) Inventeurs:
- **NADAUD, Jean-François**
  **F-75013 Paris (FR)**
- **SEBILLOTTE, Laurence**
  **F-75013 Paris (FR)**

(74) Mandataire: **Casalonga, Axel**
**BUREAU D.A. CASALONGA - JOSSE**
**Morassistrasse 8**
**D-80469 München (DE)**

(56) Documents cités:
**EP-A- 0 281 394**
**EP-A- 0 330 369**
**EP-A- 0 345 075**
**EP-A- 0 391 124**
**EP-A- 0 422 984**
**FR-A- 2 670 673**
**GB-A- 2 242 358**

EP 0 648 102 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne une composition cosmétique ou dermatologique se présentant sous forme d'une émulsion triple eau/huile de silicone/eau gélifiée, son procédé de préparation et ses applications dans le domaine cosmétique.

Depuis de nombreuses années, on utilise, notamment dans le domaine de la cosmétique, des émulsions dans les produits de traitement cosmétique de la peau. Ces émulsions sont généralement des émulsions huile-dans-eau (H/E) ou eau-dans-huile (E/H).

Des émulsions triples du type E/H/E ou encore H/E/H sont également utilisées en cosmétique ou dermatologie. De telles émulsions présentent toutefois de nombreux problèmes lors de leur fabrication ou encore des problèmes de stabilité dans le temps, et notamment lorsqu'on introduit dans ces émulsions des substances actives qui peuvent avoir tendance à déstabiliser les émulsions préparées.

La demande de brevet EP-A-0345075 (UNILEVER) décrit des émulsions triples E/H/E dans lesquelles la phase externe continue est gélifiée et qui contiennent un agent de pression osmotique dans la phase aqueuse interne, cet agent attirant l'eau de la phase externe à travers la phase huileuse. Le procédé de préparation de ces émulsions gélifiées consiste à disperser une émulsion eau-dans-huile, dont la phase aqueuse contient un agent de pression osmotique, dans une solution d'un agent gélifiant, du type polysaccharide, gélatine ou autre protéine. Ces émulsions présentent toutefois des inconvénients. La différence de pression osmotique entre la phase aqueuse interne et la phase aqueuse externe provoque une fuite d'eau de la phase aqueuse externe vers la phase aqueuse interne, donc un gonflement des globules aqueux internes, et une concentration élevée en gélifiant dans la phase aqueuse externe aboutissant à un produit trop gélatineux.

La demande de brevet EP-A-0281394 (RICHARDSON VICKS) se rapporte à une émulsion H/E/silicone. Il s'agit d'une émulsion très particulière, obtenue par introduction d'une émulsion H/E classique dans une phase huileuse siliconée qui constitue la phase huileuse externe. Cette émulsion contient différents agents tensio-actifs jouant le rôle d'émulsifiants dans l'émulsion H/E.

Or, il est connu que l'utilisation d'une quantité importante d'agents tensio-actifs dans une émulsion à usage cosmétique peut provoquer des réactions cutanées du type allergie ou irritation.

Il faut donc chercher à minimiser les quantités d'agents tensioactifs tout en ne déstabilisant pas l'émulsion.

Il est également connu que les huiles classiques utilisées dans les émulsions confèrent un toucher gras et sont souvent comédogènes.

La demanderesse a donc cherché à remplacer ces huiles classiques par des huiles ne conférant pas de toucher gras et garantissant en outre une hydratation convenable de la peau.

La demanderesse a réussi à surmonter les difficultés tant au plan technique qu'au plan cosmétique liées aux émulsions triples de l'état de la technique et à obtenir une émulsion triple possédant un aspect et un toucher tout à fait originaux ainsi que des propriétés de stabilité remarquables.

L'émulsion triple, selon l'invention, est une émulsion eau/huile de silicone/eau gélifiée (E/Si/E), dans laquelle l'émulsion primaire eau/huile de silicone est distribuée de façon homogène avec des globules de taille comprise entre 0,5 et 50 µm, ce qui lui confère un aspect lisse et brillant. L'émulsion triple selon l'invention est en outre gélifiée, translucide, onctueuse, fraîche, douce, non grasse et stable.

L'invention a donc pour objet une émulsion triple présentant les caractéristiques définies ci-après.

Un autre objet de l'invention est constitué par le procédé de préparation d'une telle émulsion.

L'invention a également pour objet l'utilisation cosmétique ou dermatologique d'une telle émulsion.

D'autres objets de l'invention apparaîtront à la lecture de la description et des exemples qui suivent.

L'émulsion triple eau/huile de silicone/eau gélifiée conforme à l'invention, est essentiellement caractérisée par le fait qu'elle comporte (A) une phase aqueuse externe continue gélifiée comprenant au moins un gélifiant à chaîne grasse du type copolymère acide ou anhydride d'acide carboxylique monoéthylénique en $C_3$-$C_6$/ester acrylique à chaîne grasse; (B) une phase grasse siliconée comprenant au moins une huile de silicone et un émulsionnant siliconé, constituant avec une phase aqueuse l'émulsion primaire E/H.

La phase aqueuse externe continue gélifiée de l'émulsion triple E/H/E selon l'invention comprend de l'eau et un gélifiant à chaîne grasse du type copolymère d'un acide carboxylique monoéthylénique comportant 3 à 6 atomes de carbone (ou son anhydride) et d'un ester acrylique à chaîne longue.

Ce type de copolymère acrylique hydrosoluble, éventuellement réticulé, qui sera appelé par la suite "gélifiant à chaîne grasse", est décrit dans EP-A-0268164. Dans ce copolymère, la proportion d'acide monomère est, de préférence, de 90 à 98% en poids et la proportion d'ester monomère est, de préférence, de 10 à 2% en poids.

L'acide monomère a pour formule :

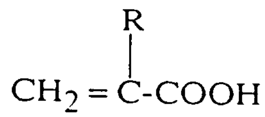

formule dans laquelle R représente H, un halogène, OH, un radical lactone ou lactame, un groupe -C≡N, ou un radical alkyle en $C_1$-$C_3$. Les monomères préférés sont l'acide acrylique et l'anhydride maléique.

L'ester monomère a pour formule :

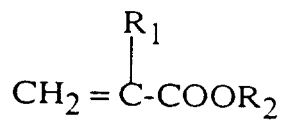

formule dans laquelle :

$R_1$ est H, méthyle ou éthyle, et

$R_2$ est un radical alkyle en $C_8$-$C_{30}$ ou oxyalkylène en $C_8$-$C_{30}$. Les radicaux alkyle en $C_{10}$-$C_{22}$ sont préférés. Parmi les esters monomères que l'on préfère, on peut citer : les acrylates et méthacrylates de décyle, lauryle, stéaryle, béhényle et mélissyle.

Les copolymères utilisés selon l'invention sont, au moins pour certains d'entre eux, distribués dans le commerce; ils sont, par exemple, commercialisés sous les dénominations PEMULEN et CARBOPOL 1342 par la Société GOODRICH.

La phase aqueuse externe continue gélifiée peut en outre comprendre d'autres constituants, tels que d'autres gélifiants du type polymères carboxyvinyliques, comme ceux vendus sous les dénominations CARBOPOL 980 ou 942 ou 950, etc., par la Société GOODRICH ou celui vendu sous la dénomination SYNTHALENK par la Société SIGMA, polyméthacrylates de glycéryle vendus par la Société GUARDIAN sous la dénomination LUBRAJEL MS, carraghénanes tels que le produit vendu par la Société SANOFI sous la dénomination SATIAGEL K80 (D. Galactopyrannose sulfate), gommes de xanthane telles que le produit xanthane/polysaccharides comprenant des unités de glucose/mannose/acide glucuronique (40/30/30), vendu par la Société KELCO sous la dénomination KELTROL.

La phase aqueuse externe peut contenir également des glycols et des neutralisants tels que la triéthanolamine ou l'hydroxyde de sodium. Elle peut aussi contenir des agents conservateurs, des colorants, des parfums, des actifs, des filtres solaires, des hydratants tels que la glycérine, des agents d'onctuosité tels que le produit SEPIGEL 305 (copolymère réticulé acrylamide/acide 2-acrylamido-2-méthylpropanesulfonique neutralisé) vendu par la Société SEPPIC.

L'émulsion primaire eau-dans-huile (E/H) comprend une phase grasse siliconée et une phase aqueuse.

La phase grasse siliconée est essentiellement constituée par au moins un émulsionnant siliconé et une huile de silicone. Elle peut comprendre en outre un épaississant choisi parmi les cires, les gommes ou les résines de silicone et éventuellement des huiles non siliconées.

Les émulsionnants siliconés contenus dans l'émulsion primaire E/H selon l'invention, peuvent être choisis parmi les diméthicone copolyols et de préférence les alkyl diméthicone copolyols de formule générale :

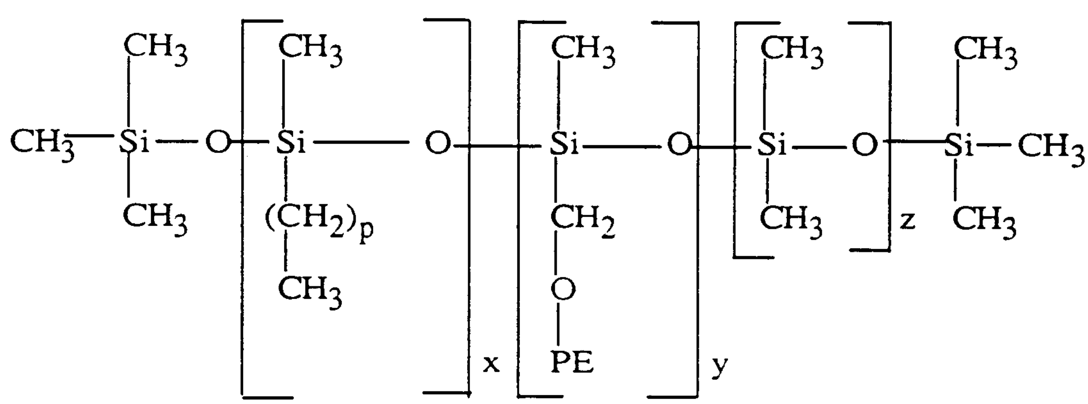

dans laquelle :

p est un nombre entier compris entre 7 et 17

x est un nombre entier compris entre 1 et 100
y est un nombre entier compris entre 1 et 40
z est un nombre entier compris entre 0 et 200
PE = $(C_2H_4O)_a$—$(C_3H_6O)_b$—H de poids moléculaire compris entre 250 et 2000;
a et b sont choisis de telle sorte que le rapport en poids des groupes $C_2H_4O/C_3H_6O$ soit compris entre 100 : 0 et 20 : 80.

On peut notamment utiliser les produits vendus sous les dénominations ABIL WE 09, ABIL EM 90 par la Société GOLDSCHMIDT, Q2 3225 C par la Société DOW CORNING et 218-1138 par la Société RHONE POULENC.

Les huiles de silicone contenues dans l'émulsion primaire, utilisables selon l'invention, peuvent être choisies parmi :

a) les cyclométhicones de formule générale :

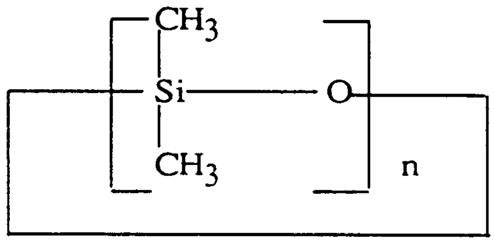

où n est un nombre entier compris entre 3 et 8.

Parmi les cyclométhicones particulièrement préférées, on peut citer le cyclotétradiméthylsiloxane (n = 4), le cyclopentadiméthylsiloxane (n = 5) et le cyclohexadiméthylsiloxane (n = 6).

On peut notamment utiliser les produits vendus par la Société DOW CORNING sous les dénominations DC FLUID 244, DC FLUID 245, DC FLUID 344 et DC FLUID 345.

D'autres cyclométhicones utilisables selon l'invention sont celles vendues par la Société GOLDSCHMIDT sous la dénomination ABIL K04, par la Société RHONE POULENC sous les dénominations SILBIONE 70045 V2 et SILBIONE HUILE 70045 V5 ainsi que par la Société UNION CARBIDE sous les dénominations VOLATIL SILICONE 7158 et VOLATIL SILICONE 7207;

b) les polydiméthylsiloxanes (DIMETHICONES) de formule générale :

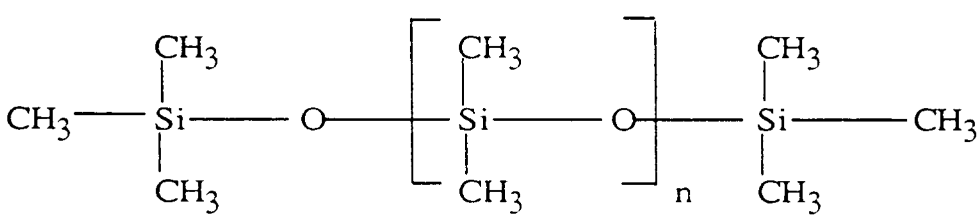

où lorsque $n < 3$, on est en présence d'une diméthicone volatile, lorsque $3 < n < 2000$, la viscosité de la diméthicone est comprise entre $1{,}5x10^{-6} m^2/s$ et $2{,}5x10^{-1}$ $m^2/s$.

Parmi les polydiméthylsiloxanes disponibles dans le commerce et utilisables dans la présente invention, on peut citer :

- DC FLUID 200 (de viscosité comprise entre $10^{-6}$ $m^2/s$ et $2{,}5x10^{-3}$ $m^2/s$) vendu par la Société DOW CORNING,
- ABIL 10 et ABIL K03 vendus par la Société GOLDSCHMIDT,
- RHODORSIL 47 V 10, SILBIONE 70047 V 10, SILBIONE 70047 V 100, SILBIONE 70047 V 300 vendus par la Société RHONE POULENC,
- SILICONE OIL L45 ($10^{-5}$ - $10^{-4}$ $m^2/s$) vendu par la Société UNION CARBIDE;

c) les polyphényltriméthylsiloxanes (PHENYLTRIMETHICONES) de formule générale :

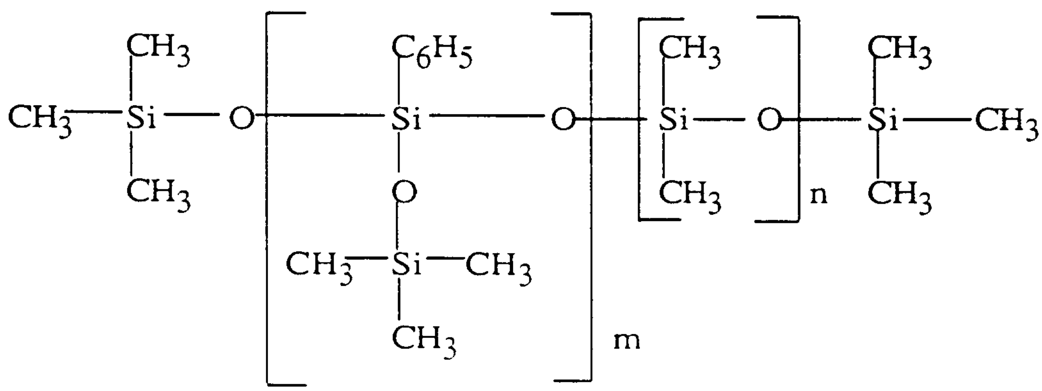

dans laquelle :
n est un nombre entier compris entre 0 et 100 et
m est un nombre entier compris entre 1 et 400.

Parmi les composés utilisables selon l'invention, on peut citer les produits commerciaux suivants : ABIL AV 200 et ABIL AV 1000 vendus par la Société GOLDSCHMIDT;
d) le monopentaérythritol triméthylsilyle de formule :

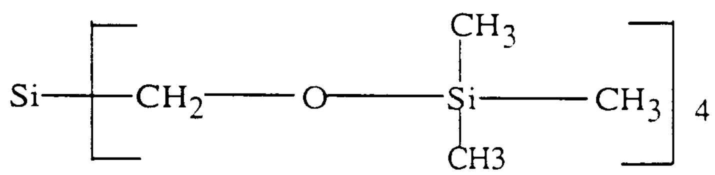

entrant dans la composition du produit vendu sous la dénomination HUILE CL 1680 par la Société RHONE POULENC;
e) des silicones fluorées, en particulier des polydiméthylsiloxanes greffés par des groupes trifluoroalkyle de formule générale :

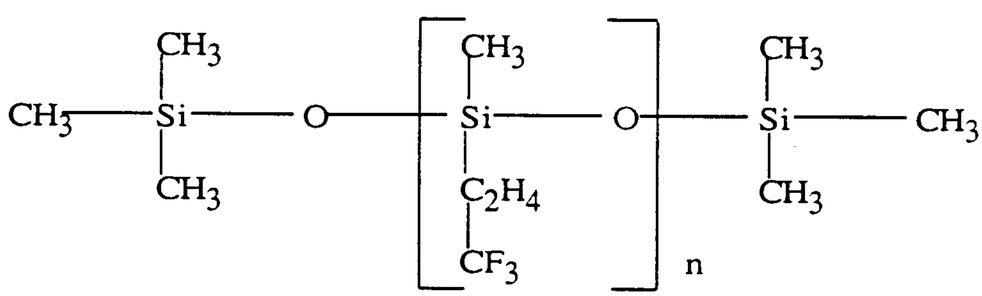

où n est un nombre entier compris entre 1 et 300, et plus particulièrement ceux vendus sous les dénominations X22-819, X22-820, X22-821, X22-822, FL-100/450 par la Société SHIN ETSU.

Les gommes de silicone utilisables dans l'émulsion primaire de la présente invention peuvent être choisies parmi :

- les polydiméthylsiloxanes (DIMETHICONES) de haut poids moléculaire de formule générale indiquée ci-dessus, dans laquelle n > 2000, tels que le produit vendu sous la dénomination VISCASIL 60M vendu par la Société GENERAL ELECTRIC;

- les polydiméthylsiloxane-OL (DIMETHICONE-OL) de formule générale :

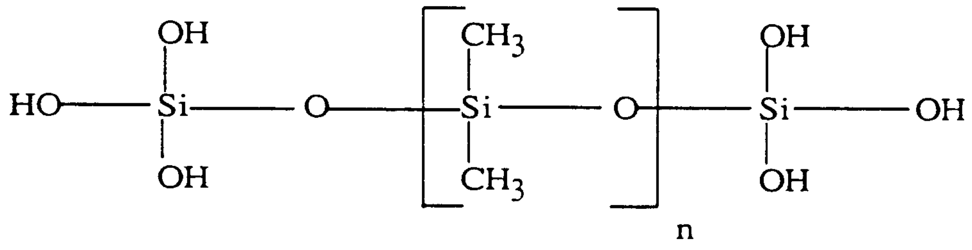

dans laquelle n > 2000,

parmi lesquelles on peut citer les produits commerciaux suivants : DC SGM-3, Q2-1403 ($10^{-4}$ $m^2/s$) et DC QC F2-1671 vendus par la Société DOW CORNING.

Parmi les cires de silicone utilisables dans l'émulsion primaire selon la présente invention, on peut citer :

- les alcoxypolydiméthylsiloxanes (ALCOXY DIMETHICONES) de formule générale :

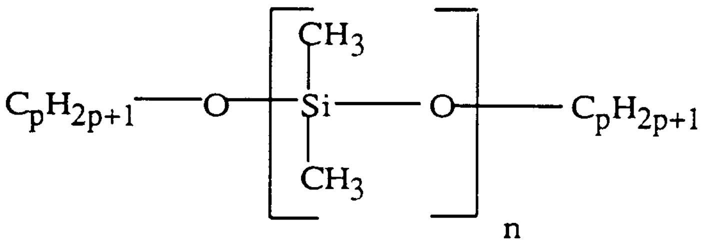

dans laquelle :
p est un nombre entier allant de 3 à 30,
n est un nombre entier allant de 1 à 200.

On peut citer plus particulièrement les stéaroxypolydiméthylsiloxanes vendus sous la dénomination ABIL WAX 2434 par la Société GOLDSCHMIDT et DC 580 vendu par la Société DOW CORNING;

- les alkylpolysiloxanes (ALKYL DIMETHICONES) de formule générale :

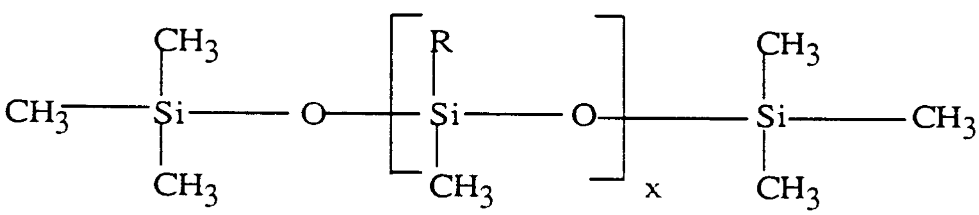

dans laquelle :
R représente $C_nH_{2n+1}$ où n est un nombre entier allant de 16 à 30,
x est un nombre entier allant de 1 à 200, tels que le produit vendu sous la dénomination 176-11187 (polycétylméthylsiloxane [$T_f$ = 32-34°C]) par la Société GENERAL ELECTRIC;

- les polydiméthylsiloxanes à fonction mercapto de formule générale :

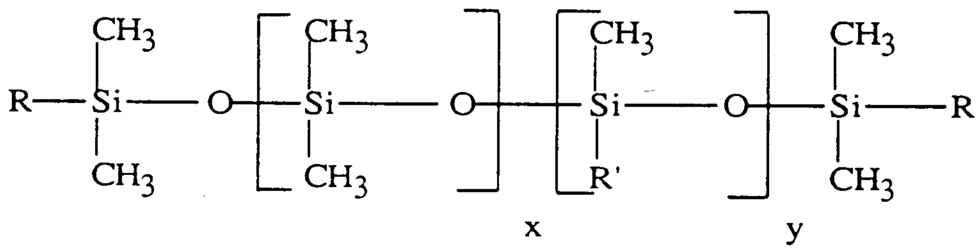

dans laquelle R et R' peuvent représenter, indépendamment l'un de l'autre, $CH_3$ ou $(CH_2)_n$ -SH, l'un au moins des radicaux R et R' étant $(CH_2)_n$ - SH, n étant supérieur ou égal à 3 et de préférence inférieur à 30,
x est un nombre entier compris entre 10 et 300 ,
y est un nombre entier compris entre 1 et 40, tels que le produit vendu sous la dénomination EXP-77 MERCAPTO FONCTIONAL SILICONE WAX par la Société GENESEE (copolymère mercaptosilicone).

Les résines de silicone utilisables dans l'émulsion primaire de la présente invention peuvent être choisies parmi les triméthylsiloxysilicates de formule générale :

$$[(CH_3)_3—Si—O_{1/2}]_x[SiO_2]_y$$

parmi lesquels on peut citer les produits vendus sous la dénomination DC FLUID 593 par la Société DOW CORNING, et les produits dénommés SILICONE SSX 4267, SILICONE SS 4230 et SILICONE SS 4267, vendus par la Société GENERAL ELECTRIC.

Les huiles non siliconées utilisables dans la phase grasse de l'émulsion primaire de la présente invention sont choisies parmi les huiles fluides, stables, pharmaceutiquement ou cosmétiquement acceptables, et leurs mélanges.

Ces huiles sont choisies parmi les huiles végétales ou animales, modifiées ou non, telles que l'huile d'amande douce, l'huile d'avocat, l'huile de ricin, l'huile d'olive, l'huile de jojoba, l'huile de tournesol, l'huile de germe de blé, l'huile de sésame, l'huile d'arachide, l'huile de pépins de raisin, l'huile de soja, l'huile de colza, l'huile de carthame, l'huile de coprah, l'huile de mais, l'huile de noisette, le beurre de karité, l'huile de palme, l'huile de noyau d'abricot, l'huile de calophyllum, l'huile de purcellin; les huiles minérales telles que par exemple l'huile de vaseline; les huiles synthétiques et les triglycérides d'acides gras tels que les triglycérides caprylique/caprique, les triglycérides en $C_{10}$ à $C_{18}$.

La phase grasse de l'émulsion primaire E/H peut en outre contenir des additifs lipophiles habituellement utilisés en cosmétique, par exemple des filtres solaires.

La phase aqueuse de l'émulsion primaire E/H comprend de l'eau, un hydratant tel que la glycérine, un polyol tel que le propylèneglycol, l'hexylèneglycol, le dipropylèneglycol, le 1,3-butylèneglycol et un agent de stabilisation de l'émulsion, par exemple le chlorure de sodium, le dichlorure de magnésium et le sulfate de magnésium. Elle peut aussi contenir les additifs habituels tels que conservateurs, parfums, colorants, actifs et filtres solaires.

La demanderesse a constaté que l'ajout dans l'émulsion primaire E/H de composés comportant des chaînons organofluorés et des chaînons hydrocarbonés, permet de diminuer l'effet collant provoqué par le gélifiant à chaîne grasse contenu dans la phase aqueuse externe, et permet d'apporter plus de douceur et d'onctuosité au produit fini.

Ces composés comportant des chaînons organofluorés et des chaînons hydrocarbonés, ont une structure chimique comportant un squelette carboné où certains atomes d'hydrogène ont été substitués par des atomes de fluor, le squelette carboné pouvant comporter un ou plusieurs hétéroatomes et un ou plusieurs groupements organiques fonctionnels.

Pour les composés comportant des chaînon organofluorés et des chaînons hydrocarbonés, on définit le taux de substitution des atomes d'hydrogène par des atomes de fluor, sous la forme du rapport : nombre d'atomes de fluor/(nombre d'atomes de fluor + nombre d'atomes d'hydrogène) où seuls les atomes d'hydrogène liés aux atomes de carbone du squelette sont pris en compte. Les composés comportant des chaînon organofluorés et des chaînons hydrocarbonés utilisés dans les émulsions selon l'invention, comportent au moins un groupement hydrocarboné dans la molécule.

Ces composés comportant des chaînons organoiluorés utilisés selon l'invention, ont de préférence un taux de substitution compris entre 0,5 et 95%. De façon préférée, ce taux est supérieur à 10% et inférieur à 80%.

Les composés comportant des chaînons organofluorés et des chaînons hydrocarbonés utilisés selon la présente invention, ont la formule suivante :

$$(R_F)_x - (A)_y - (R_H)_z$$

dans laquelle :

x représente 1, 2 ou 3,

y représente 0 ou 1,

z représente 0, 1, 2 ou 3,

à la condition que y et z ne soient pas simultanément 0, et que lorsque z est 0, x est 2 ou 3.

$R_F$ représente un radical fluoré aliphatique ou aromatique, saturé ou insaturé, à chaîne linéaire, ramifiée ou cyclique, cette chaîne pouvant être fonctionnalisée et/ou être interrompue par des atomes divalents tels que l'oxygène ou le soufre, ou trivalents tels que l'azote, et/ou substituée par des atomes d'hydrogène ou d'autres atomes d'halogène, à la condition que, pour deux atomes de carbone de la chaîne, ne soit pas présent plus d'un de ces substituants autres que le fluor.

$R_H$ représente un radical hydrocarboné aliphatique ou aromatique, saturé ou insaturé, à chaîne linéaire, ramifiée ou cyclique, cette chaîne pouvant être fonctionnalisée et/ou interrompue par un ou plusieurs atomes divalents tels que l'oxygène ou le soufre ou par un ou plusieurs atomes trivalents comme l'azote.

A représente un radical di-, tri- ou quadrivalent, tel que :

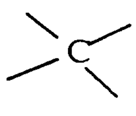 , 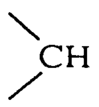 , 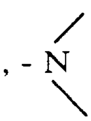 , 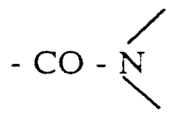 , 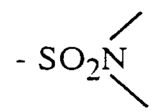 , 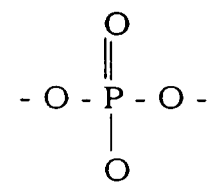

ainsi que les structures cycliques, aliphatiques ou aromatiques comprenant un tel radical, ou des insaturations éthyléniques.

Par l'expression "fonctionnalisé", on entend selon l'invention, une substitution du squelette intercalaire, terminale ou pendante, par au moins un groupement organique fonctionnel comme une fonction alcool, thiol, acide, carbonyle, sulfoxyde, ester, amide, amine, phosphate, éthylénique, acétylénique, et énamine ou sulfonamide.

Par insaturation éthylénique, on entend par exemple :

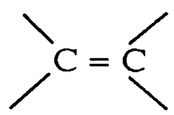 , 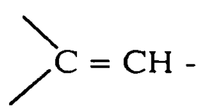

ou - CH = CH -

De préférence, $R_H$ représente un radical alkyle linéaire ou ramifié en $C_1$-$C_{22}$ ou un mélange de radicaux alkyle linéaires ou ramifiés en $C_1$-$C_{22}$, un radical aryle en $C_6$-$C_{10}$ ou un radical aralkyle en $C_7$-$C_{15}$.

De préférence, $R_F$ représente un radical perfluoroalkyle ayant de 4 à 22 atomes de carbone.

A titre illustratif, on peut citer les composés possédant des groupes perfluorocarbonés et des groupes hydrocarbonés, le nombre total d'atomes de carbone étant compris entre 10 et 30, le nombre d'atomes de carbone des groupes hydrocarbonés étant égal ou supérieur à deux fois le nombre d'atomes de carbone des groupes perfluorocarbonés tels qu'ils sont décrits dans le document JP 63-002916.

De même, à titre illustratif, on peut citer les composés organofluorés hydrocarbonés dont la structure générale est définie par la formule :

$$R_1 - (CH_2)_n - X - [C_3H_5 (OH)] - (Y)_x - R_2$$

où $C_3H_5$ (OH) représente :

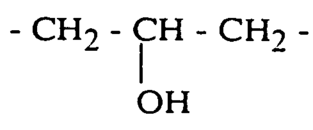 ou 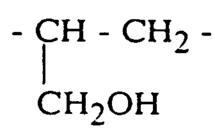 ou 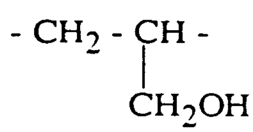

$R_1$ représente un radical alkyle perfluoré, linéaire ou ramifié en $C_4$-$C_{20}$ ou un mélange de radicaux perfluorés, linéaires ou ramifiés en $C_4$-$C_{20}$,

$R_2$ représente un radical alkyle linéaire ou ramifié en $C_1$-$C_{22}$ ou un mélange de radicaux alkyle, linéaires ou

ramifiés en $C_1$-$C_{22}$ ou un radical aryle en $C_6$-$C_{10}$ ou aralkyle en $C_7$-$C_{15}$,

X et Y, identiques ou différents, représentent :

- O - , - S -,

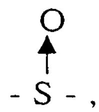 ou 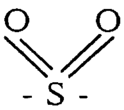 ;

sous réserve que X et Y ne représentent pas simultanément

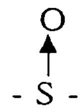 ou 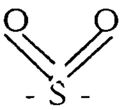 ,

n est compris entre 0 et 4, et

x représente 0 ou 1.

Ces composés utilisés selon l'invention sont décrits dans FR-A 2.684.668 et EP-A-166.696.

Par ailleurs, on peut également utiliser, selon l'invention, les composés de formule :

$$R_F - (CH_2)_n - X - [C_3H_5\,(OH)] - Y - (CH_2)_m - R'_F$$

dans laquelle $C_3H_5$ (OH) représente les structures :

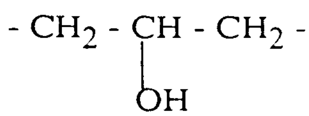 ou 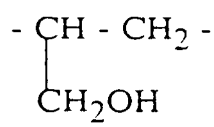 ou 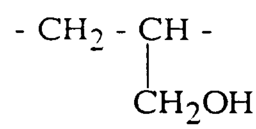

$R_F$ et $R'_F$, identiques ou différents, représentent un radical alkyle perfluoré, linéaire ou ramifié en $C_4$-$C_{20}$ ou un mélange de radicaux alkyle perfluorés, linéaires ou ramifiés en $C_4$-$C_{20}$;

m et n, identiques ou différents, représentent 0, 1, 2, 3 ou 4;

X et Y, identiques, sont - O - ou - S - .

Ces composés sont décrits dans DE-2.702.607, JP 89-193.236, JP 92-275.268 et US-3.893.984.

On peut aussi utiliser, selon l'invention, les composés décrits dans le document US-3.952.066, de formule :

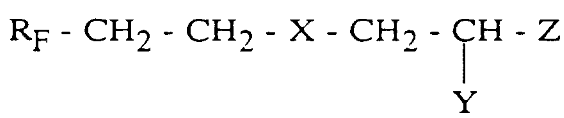

où Y est OH, et

Z est

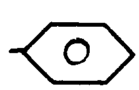 , -

- $CH_3$, - $CH_2OH$, - $CH_2$ O $COCH_3$

ou bien Y est - $CH_2OH$ et Z est - O-$COCH_3$

X représente - O-, - S - ,

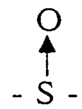 ou 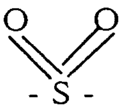 ,

et

$R_F$ représente un radical alkyle perfluoré, linéaire ou ramifié, en $C_4$-$C_{20}$, ou un mélange de radicaux alkyle perfluorés, linéaires ou ramifiés, en $C_4$-$C_{20}$ ;

ou bien les composés décrits dans le document DE 2.052.579, de formule :

$$R_F - CH = CH - CH_2 - O - CH_2 - [C_2H_4 - OW]$$

où

$C_2H_4OW$ désigne :

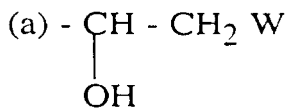 ou 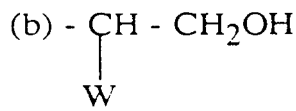

W désignant : -OR, -SR, -COOR,

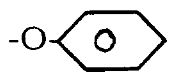 , 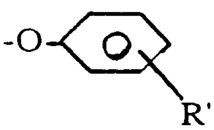

R désigne un radical alkyle en $C_1$-$C_{18}$, linéaire ou ramifié,

R' désigne -$CH_3$ ou -OH, en position ortho ou para, et

$R_F$ représente un radical alkyle perfluoré, linéaire ou ramifié, en $C_4$-$C_{20}$, ou un mélange de radicaux alkyle perfluorés, linéaires ou ramifiés, en $C_4$-$C_{20}$.

Selon un premier mode de réalisation de l'invention, au moins une huile de silicone est utilisée en tant que phase grasse pour la préparation de l'émulsion primaire E/H.

Selon un second mode de réalisation de l'invention, une telle huile de silicone est ajoutée en tant que produit de dilution à l'émulsion primaire E/H contenant, à titre de phase grasse, au moins une huile siliconée ou non siliconée, juste avant la dispersion de l'émulsion primaire dans la phase aqueuse externe gélifiée, pour obtenir l'émulsion triple finale stable.

L'émulsion triple de la présente invention contient 0,05 à 8% en poids d'émulsionnant siliconé, 0,25 à 60% en poids d'huile de silicone, 0 à 16% en poids de gomme de silicone, 0 à 16% en poids de cire de silicone, 0 à 8% en poids de résine de silicone, 0 à 25% en poids de composé à chaînons organofluorés et hydrocarbonés, 0 à 50% en poids de glycols, 0,1 à 3% en poids de gélifiant à chaîne grasse, 0,1 à 3% en poids de neutralisant (triéthanolamine, hydroxyde de sodium), ainsi que les agents de stabilisation de l'émulsion primaire indiqués ci-dessus, et éventuellement des substances actives cosmétologiques ou dermatologiques, ainsi que d'autres adjuvants habituellement utilisés en cosmétique tels que des agents conservateurs, des colorants, des parfums, des hydratants, des filtres solaires, le complément étant constitué par de l'eau.

L'invention a également pour objet le procédé de préparation d'une émulsion triple selon l'invention.

Selon un mode de réalisation préféré du procédé de l'invention, on prépare, dans un premier temps, l'émulsion primaire eau-dans-huile en ajoutant une phase aqueuse à la phase grasse pour l'obtention d'une émulsion E/H, l'usage d'une quantité maximale de 10% en poids d'huile de silicone dans l'émulsion primaire, calculée par rapport au poids total de l'émulsion triple, permettant de préparer une émulsion stable à température ambiante.

Dans un deuxième temps, on peut, le cas échéant, diluer l'émulsion primaire ainsi obtenue, avant sa dispersion dans la phase aqueuse gélifiée, avec une huile de silicone qui peut être identique à ou différente de celle(s) utilisée(s) dans l'émulsion primaire.

L'émulsion primaire est préparée de manière à obtenir une viscosité de l'émulsion, dans sa forme diluée ou non, comprise entre 0,1 Pa.s et 3 Pa.s, de préférence entre 0,13 Pa.s et 1,1 Pa.s.

Dans un troisième temps, on réalise l'émulsion triple par addition de l'émulsion primaire ainsi obtenue ou du produit dilué avec une huile de silicone, à une seconde phase aqueuse gélifiée qui constitue la phase aqueuse externe de l'émulsion, de manière à obtenir un rapport R des proportions dans l'émulsion triple (ET) de l'émulsionnant siliconé de l'émulsion primaire (EP) et du gélifiant à chaîne grasse de la phase aqueuse externe compris entre 0,1 et 1,75 et de préférence entre 0,2 et 1,2 :

$$R = \frac{\%(g)\ \text{émulsionnant siliconé de EP}}{\%(g)\ \text{gélifiant à chaîne grasse de la phase aqueuse externe}}$$

Comme déjà indiqué ci-dessus, les compositions sous forme d'émulsion triple conformes à l'invention, présentent des propriétés cosmétiques particulièrement remarquables, notamment au niveau du toucher et de l'aspect, ce qui permet de les utiliser comme bases pour appliquer les substances actives cosmétiques sur la peau.

L'introduction dans la phase aqueuse interne et/ou externe et/ou dans la phase huileuse de substances actives, permet de nombreuses utilisations d'une telle émulsion triple.

Ces émulsions peuvent notamment être utilisées dans des produits de soin du visage pour peaux sèches ou peaux grasses. Pour la réalisation de produits pour peaux sèches, on peut introduire dans l'une quelconque des deux phases aqueuses, des substances actives hydrosolubles hydratantes telles que par exemple la glycérine, le propylèneglycol, le sorbitol, la proline, l'acide pyrrolidone carboxylique et ses dérivés, l'urée, le collagène hydrolysé, le gel d'Aloe Vera, l'acide hyaluronique et ses dérivés, le hyaluronate de diméthylsilanol et l'allantoïne.

Des produits de traitement pour peaux grasses sont obtenus en introduisant dans l'une quelconque des deux phases aqueuses, des substances actives hydrosolubles telles que la provitamine B5 qui est utilisée comme adoucissant ou un antibactérien tel que le transthiolane diol 3,4-S-dioxyde.

Les émulsions selon l'invention peuvent également être utilisées comme produits démaquillants ou de nettoyage pour le visage sous forme de crèmes, de laits ou de masques par exemple, ou comme produits de maquillage par incorporation de charges ou de pigments.

Ces émulsions peuvent aussi être utilisées comme produits solaires par introduction de filtres.

Comme filtres solaires hydrosolubles pouvant être introduits dans la phase aqueuse interne et/ou externe, on peut utiliser par exemple l'acide 2-hydroxy 4-méthoxy benzophénone 5-sulfonique vendu sous la dénomination UVINUL MS 40 par la Société BASF. Comme filtres solaires liposolubles à introduire dans la phase huileuse, on peut utiliser par exemple le paraméthoxycinamate de 2-éthylhexyle vendu sous la dénomination PARSOL MCX par la Société GIVAUDAN ou la 2-hydroxy 4-méthoxybenzophénone vendue sous la dénomination UVINUL M 40 par la Société BASF.

Les émulsions selon l'invention peuvent également être utilisées dans la préparation de produits après-solaires contenant par exemple en tant qu'agents actifs apaisants, la vitamine F et les hydratants cités plus haut.

On peut aussi, en introduisant des substances actives amincissantes hydrosolubles ou liposolubles respectivement dans l'une des deux phases aqueuses ou dans la phase grasse, obtenir des produits amincissants.

Parmi les substances actives amincissantes hydrosolubles, on peut citer les dérivés de xanthine tels que la caféine, la théobromine, la théophylline, la L-carnitine, le chlorhydrate de diméthylaminoéthyl théophylline, les dérivés de silicium du type méthylsilanol théophyllinacétatealginate ou des dérivés végétaux tels que des extraits hydroglycoliques de lierre grimpant, d'algue brune, de pensée sauvage fraîche. Parmi les substances actives amincissantes liposolubles, on peut citer le nicotinate de DL-alpha-tocophérol, l'extrait huileux de racine de ginseng (Panax ginseng), l'extrait huileux de lierre grimpant (Hedera Helix), l'extrait huileux de fleurs sèches d'arnica (Arnica Montana L), l'extrait huileux d'algue (Fucus Vesiculosus).

On peut également obtenir des produits de soin corporel en introduisant des hydratants cités précédemment et des huiles végétales ou minérales, ainsi que des produits capillaires utilisés par exemple pour lisser les cheveux en introduisant des filtres solaires pour protéger les cheveux du rayonnement UV.

Les émulsions triples de l'invention peuvent également être utilisées comme produits pour jambes lourdes contenant comme actifs hydrosolubles le ginkgo biloba, le mélilot ou le ruscus et des huiles classiques comme émollients.

Les exemples suivants sont destinés à illustrer l'invention sans pour autant présenter un caractère limitatif.

EXEMPLES DE FORMULATION

EXEMPLE 1

EMULSION TRIPLE AMINCISSANTE

**PHASE A (E/H)**

| | | |
|---|---|---|
| * ABIL WE 09 R (tensio-actif siliconé à 66,6% de MA) | | 0,5 g |
| * DC QC F2 1671 R (gomme de silicone) (Société DOW CORNING) | | 0,8 g |
| * VOLATIL SILICONE 7158 R (huile de silicone) | | 2,7 g |
| * Caféine (Société PROLABO) | | 0,5 g |
| - Chlorure de sodium | | 0,16 g |
| * Glycérine | | 0,4 g |
| * Propylèneglycol | | 4 g |
| - Conservateurs, parfums, colorants | qs | |
| - Eau distillée | | 4 g |

**PHASE B**

| | | |
|---|---|---|
| * CARBOPOL 1342 R (gélifiant à chaîne grasse) | | 0,3 g |
| * CARBOPOL 980 R (gélifiant) | | 0,3 g |
| * Glycérine | | 2 g |
| * Propylèneglycol | | 20 g |
| - Conservateurs, colorants | qs | |
| * Triéthanolamine à 99% | | 0,6 g |
| - Eau distillée | qsp | 100 g |

$$R = \frac{0,5 \times 66,6\%}{0,3} = 1,1$$

* cf annexe décrivant les matières premières

On prépare l'émulsion triple en introduisant la phase A dans la phase B et en terminant par la neutralisation avec la triéthanolamine.

On obtient une crème amincissante d'aspect gélifié, translucide, qui permet le massage.

EXEMPLE 2

EMULSION TRIPLE POUR LES MAINS

**PHASE A (E/H)**

| | | |
|---|---|---|
| * DC Q2 3225C R (tensio-actif siliconé à 10% de MA) | 5 | g |
| * DC FLUID 200 R (huile de silicone) (Société DOW CORNING) | 0,4 | g |
| * DC FLUID 593 R (résine de silicone) (Société DOW CORNING) | 1 | g |
| * Glycérine | 7 | g |
| - Sulfate de magnésium | 0,4 | g |
| - Eau distillée | 6,2 | g |

**PHASE B**

| | | |
|---|---|---|
| * VOLATIL SILICONE 7158 R (huile de silicone) | 10 | g |

**PHASE C**

| | | |
|---|---|---|
| * CARBOPOL 1342 R (gélifiant à chaîne grasse) | 0,3 | g |
| * CARBOPOL 980 R (gélifiant) | 0,3 | g |
| * NIPASTAT R (conservateurs) | 0,06 | g |
| * Glycérine | 2 | g |
| * GERMALL 115 R | 0,2 | g |
| * Triéthanolamine à 99% | 0,6 | g |
| - Eau distillée qsp | 100 | g |

$$R = \frac{0,5}{0,3} = 1,7$$

La phase A est diluée avec la phase B, puis on disperse l'ensemble dans la phase C pour obtenir l'émulsion triple finale.

On obtient un gel-crème blanc légèrement translucide, lisse et brillant, ayant des propriétés hydratantes.

EXEMPLE 3

EMULSION TRIPLE POUR PEAUX GRASSES

**PHASE A (E/H)**

| | | |
|---|---|---|
| * ABIL WE 09 R (tensio-actif siliconé à 66,6% de MA) | 0,6 | g |
| * VISCASIL 60 M R (gomme de silicone) (Société GENERAL ELECTRIC) | 0,2 | g |
| * ABIL K 03 R (huile de silicone) (Société GOLDSCHMIDT) | 1 | g |
| * DC FLUID 345 R (huile de silicone) | 3,4 | g |
| * Glycérine | 2 | g |
| - Sulfate de magnésium | 0,2 | g |
| - MEXORYL SN R (antibactérien) (Société CHIMEX) | 0,5 | g |
| - Eau distillée | 11,1 | g |

**PHASE B**

| | | |
|---|---|---|
| * DC FLUID 345 R (huile de silicone) | 5 | g |

**PHASE C**

| | | | |
|---|---|---|---|
| * CARBOPOL 1342 R (gélifiant à chaîne grasse) | | 0,6 | g |
| * Glycérine | | 5 | g |
| * Propylèneglycol | | 10 | g |
| - Conservateurs, colorants | qs | | |
| * Triéthanolamine à 99% | | 0,6 | g |
| - Eau distillée | qsp | 100 | g |

$$R = \frac{0,6 \times 66,6\%}{0,6} = 0,7$$

Comme dans l'exemple précédent, on dilue la phase A avec la phase B avant de disperser l'ensemble dans la phase C.

On obtient un gel-crème translucide, non gras, qui s'étale et pénètre bien, adapté aux peaux grasses.

EXEMPLE 4

## EMULSION TRIPLE TEINTEE POUR LE VISAGE

**PHASE A (E/H)**

| | | |
|---|---|---|
| * ABIL WE 09 $^R$ (tensio-actif siliconé à 66,6% de MA) | | 0,5 g |
| * DC FLUID 200 $^R$ (huile de silicone) | | 0,5 g |
| * VOLATIL SILICONE 7158 $^R$ (huile de silicone) | | 1,3 g |
| * DC Q2 1403 FLUID $^R$ (gomme de silicone) | | 0,4 g |
| - Pigments | | 0,14 g |
| - Talc | | 0,2 g |
| * Glycérine | | 0,8 g |
| * Propylèneglycol | | 1 g |
| - Polyéthylèneglycol | | 0,3 g |
| - Eau distillée | | 4,86 g |

**PHASE B**

| | | |
|---|---|---|
| * VOLATIL SILICONE 7158 $^R$ (huile de silicone) | | 15 g |

**PHASE C**

| | | |
|---|---|---|
| * CARBOPOL 1342 $^R$ (gélifiant à chaîne grasse) | | 0,6 g |
| * Triéthanolamine à 99% | | 0,6 g |
| - Eau distillée | qsp | 100 g |

$$R = \frac{0,5 \times 66,6\%}{0,6} = 0,6$$

Pour préparer l'émulsion triple, on procède comme dans l'exemple précédent.

On obtient une crème teintée gélifiée, agréable à appliquer.

EXEMPLE 5

EMULSION TRIPLE SOLAIRE

**PHASE A (E/H)**

| | | |
|---|---|---|
| * ABIL WE 09 R (tensio-actif siliconé à 66,6% de MA) | | 0,8 g |
| * VOLATIL SILICONE 7158 R (huile de silicone) | | 2,8 g |
| * CB 185 R (filtre UV) (Société RHONE POULENC) | | 0,5 g |
| * ABIL AV 1000 R (huile de silicone) (Société GOLDSCHMIDT) | | 0,6 g |
| * ABIL WAX 2434 R (cire de silicone) | | 0,6 g |
| - VITAMINE E ACETATE (Société HOFFMANN LAROCHE) | | 0,4 g |
| * Glycérine | | 0,4 g |
| * Propylèneglycol | | 4 g |
| - Chlorure de sodium | | 0,16 g |
| - Conservateurs, colorants | qs | |
| - Eau distillée | qsp | 100 g |

**PHASE B**

| | |
|---|---|
| * VOLATIL SILICONE 7158 R (huile de silicone) | 10 g |

**PHASE C**

| | | |
|---|---|---|
| * CARBOPOL 1342 R (gélifiant à chaîne grasse) | | 0,8 g |
| * Propylèneglycol | | 20 g |
| - Conservateurs, parfums | qs | |
| * Triéthanolamine à 99% | | 0,8 g |
| - Eau distillée | qsp | 100 g |

$$R = \frac{0,8 \times 66,6\%}{0,8} = 0,7$$

On procède comme dans l'exemple précédent pour préparer cette émulsion triple.

On obtient une crème gélifiée protectrice anti-UV.

EXEMPLE 6

EMULSION TRIPLE-BAUME GAINANT APRES-SHAMPOOING

**PHASE A (E/H)**

| | | | |
|---|---|---|---|
| * ABIL WE 09 R (tensio-actif siliconé à 66,6% de MA) | | 0,8 | g |
| * VOLATIL SILICONE 7158 R (huile de silicone) | | 2,8 | g |
| * HUILE CL 1680 R (huile de silicone) (Société RHONE POULENC) | | 0,4 | g |
| * VISCASIL 60 M R (gomme de silicone) | | 0,2 | g |
| - Chlorure de sodium | | 0,16 | g |
| - Conservateurs | qs | | |
| * Glycérine | | 0,2 | g |
| - Eau distillée | qsp | 100 | g |

**PHASE B**

| | | | |
|---|---|---|---|
| * VOLATIL SILICONE 7158 R (huile de silicone) | | 5 | g |

**PHASE C**

| | | | |
|---|---|---|---|
| * PEMULEN TR 1 R (Gélifiant à chaîne grasse) (Société GOODRICH) | | 1 | g |
| * Triéthanolamine à 99% | | 1 | g |
| - Eau distillée | qsp | 100 | g |

$$R = \frac{0{,}8 \times 66{,}6\%}{1} = 0{,}5$$

On procède comme dans l'exemple précédent pour préparer l'émulsion triple.

On obtient un gel blanc qui permet la protection et le gainage des cheveux.

EXEMPLE 7

EMULSION TRIPLE GEL CORPOREL

**PHASE A (E/H)**

| | | | |
|---|---|---|---|
| * ABIL WE 09 R (tensio-actif siliconé à 66,6% de MA) | | 0,8 | g |
| * VOLATIL SILICONE 7158 R (huile de silicone) | | 2,6 | g |
| * FL 100/450 R (huile de silicone fluorée) (Société SHIN ETSU) | | 1,4 | g |
| - Parfums | qs | | |
| - Chlorure de sodium | | 0,16 | g |
| * Propylèneglycol | | 4 | g |
| * Glycérine | | 0,4 | g |
| - Conservateurs | qs | | |
| - Eau distillée | | 10,64 | g |

**PHASE B**

| | | | |
|---|---|---|---|
| * PEMULEN TR 1 R (gélifiant à chaîne grasse) | | 0,8 | g |
| - Butylèneglycol | | 10 | g |
| * Glycérine | | 5 | g |
| * Triéthanolamine à 99% | | 0,8 | g |
| - Conservateurs, colorants | qs | | |
| - Eau distillée | qsp | 100 | g |

$$R = \frac{0{,}8 \times 66{,}6\%}{0{,}8} = 0{,}7$$

Pour préparer l'émulsion triple, on prépare la phase A que l'on disperse dans la phase B.

On obtient un gel-crème translucide, onctueux et brillant, frais à l'application et ayant des propriétés hydratantes et émollientes.

EXEMPLE 8

EMULSION TRIPLE-GEL POUR LE CORPS

**PHASE A (E/H)**

| | |
|---|---|
| * ABIL WE 09R (tensio-actif siliconé à 66,6% de MA) | 0,6 g |
| * VOLATIL SILICONE 7158R (huile de silicone) | 1,5 g |
| - 1-(2'-F-hexyléthylthio)-3-(2"-éthyl-hexyloxy)-2-propanol | 1,5 g |
| * Propylèneglycol | 3 g |
| - Chlorure de sodium | 0,12 g |
| - Conservateurs | 0,03 g |
| - Eau distillée | 8,25 g |

**PHASE B**

| | | |
|---|---|---|
| * CARBOPOL 1342 R (gélifiant à chaîne grasse) | | 0,6 g |
| * Propylèneglycol | | 20 g |
| * Glycérine | | 3 g |
| - Conservateurs, parfums | | 0,26 g |
| * Triéthanolamine à 99% | | 0,2 g |
| - Eau distillée | qsp | 100 g |

$$R = \frac{0,6 \times 66,6\%}{0,6} = 0,66$$

Pour préparer l'émulsion triple, on procède comme dans l'exemple 1.

On obtient un gel lisse, translucide, brillant, agréable à appliquer.

EXEMPLE 9

EMULSION TRIPLE-GEL POUR LE CORPS

**PHASE A (E/H)**

| | |
|---|---|
| * ABIL WE 09[R] (tensio-actif siliconé à 66,6% de MA) | 0,8 g |
| * VOLATIL SILICONE 7158[R] (huile de silicone) | 2 g |
| - 1-(2'-F-hexyléthylthio)-3-(2"-éthyl-hexyloxy)-2-propanol | 2 g |
| * Propylèneglycol | 4 g |
| - Chlorure de sodium | 0,16 g |
| - Conservateurs | 0,04 g |
| - Eau distillée | 11 g |

**PHASE B**

| | | |
|---|---|---|
| * CARBOPOL 1342[R] (gélifiant à chaîne grasse) | | 0,6 g |
| * Propylèneglycol | | 20 g |
| * Glycérine | | 2 g |
| - Conservateurs, parfums | | 0,26 g |
| * Triéthanolamine à 99% | | 0,6 g |
| - Eau distillée | qsp | 100 g |

$$R = \frac{0,8 \times 66,6\%}{0,6} = 0,88$$

Pour préparer l'émulsion triple, on procède comme dans l'exemple 1.

On obtient une composition gélifiée, translucide et brillante, agréable à appliquer.

EXEMPLE 10

EMULSION TRIPLE POUR LE CORPS

**PHASE A (E/H)**

| | |
|---|---|
| * ABIL WE 09[R] (tensio-actif siliconé à 66,6% de MA) | 1 g |
| * VOLATIL SILICONE 7158[R] (huile de silicone) | 2,5 g |
| - 1-(2'-F-hexyléthylthio)-3-(2"-éthyl-hexyloxy)-2-propanol | 2,5 g |
| * Propylèneglycol | 5 g |
| - Chlorure de sodium | 0,2 g |
| - Conservateurs | 0,05 g |
| - Eau distillée | 13,75 g |

**PHASE B**

| | | |
|---|---|---|
| * CARBOPOL 1342 [R] (gélifiant à chaîne grasse) | | 0,6 g |
| * Propylèneglycol | | 20 g |
| * Glycérine | | 2 g |
| - Conservateurs, parfums | | 0,26 g |
| * Triéthanolamine à 99% | | 0,2 g |
| - Eau distillée | qsp | 100 g |

$$R = \frac{1 \times 66{,}6\%}{0{,}6} = 1{,}1$$

Pour préparer l'émulsion, on procède comme dans l'exemple 1.

On obtient un gel lisse, translucide, brillant, agréable à appliquer.

EXEMPLE 11

EMULSION TRIPLE POUR LE VISAGE

**PHASE A (E/H)**

| | | |
|---|---|---|
| * Cétyldiméthicone copolyol, vendu sous la dénomination ABIL EM90 par la Société GOLDSCHMIDT (tensio-actif siliconé) | 0,45 | g |
| * Glycoldistéarate + Tristéarin, vendu sous la dénomination UNITWIX par la Société GUARDIAN | 0,075 | g |
| - 1-(2'-F-hexyléthylthio)-3-(2"-éthyl-hexyloxy)-2-propanol | 3,6 | g |
| * Glycérine | 0,75 | g |
| - Sulfate de magnésium | 0,105 | g |
| - Conservateurs | 0,045 | g |
| - Eau distillée | 9,975 | g |

**PHASE B**

| | | |
|---|---|---|
| * VOLATIL SILICONE 7158 R (huile de silicone) | 6 | g |

**PHASE C**

| | | | |
|---|---|---|---|
| * CARBOPOL 1342 R (gélifiant à chaîne grasse) | | 0,6 | g |
| * Propylèneglycol | | 20 | g |
| * Glycérine | | 2 | g |
| - Conservateurs, parfum | | 0,26 | g |
| * Triéthanolamine à 99% | | 0,6 | g |
| - Eau distillée | qsp | 100 | g |

$$R = \frac{0,45}{0,6} = 0,75$$

Pour préparer l'émulsion triple, on procède comme dans l'exemple 2.

On obtient un gel translucide, agréable à appliquer.

EXEMPLE 12

EMULSION TRIPLE POUR LE VISAGE

**PHASE A (E/H)**

| | | |
|---|---|---|
| * Cétyldiméthicone copolyol, vendu sous la dénomination ABIL EM90 par la Société GOLDSCHMIDT (tensio-actif siliconé) | 0,6 | g |
| * Glycoldistéarate + Tristéarin, vendu sous la dénomination UNITWIX par la Société GUARDIAN | 0,1 | g |
| - 1-(2'-F-hexyléthylthio)-3-(2"-éthyl-hexyloxy)-2-propanol | 4,8 | g |
| * Glycérine | 1 | g |
| - Sulfate de magnésium | 0,14 | g |
| - Conservateurs | 0,06 | g |
| - Eau distillée | 13,3 | g |

**PHASE B**

| | | |
|---|---|---|
| * VOLATIL SILICONE 7158 R (huile de silicone) | 6 | g |

**PHASE C**

| | | |
|---|---|---|
| * CARBOPOL 1342 R (gélifiant à chaîne grasse) | 0,6 | g |
| * Propylèneglycol | 20 | g |
| * Glycérine | 2 | g |
| - Conservateurs, parfum | 0,26 | g |
| * Triéthanolamine à 99% | 0,6 | g |
| - Eau distillée qsp | 100 | g |

$$R = \frac{0,6}{0,6} = 1$$

Pour préparer l'émulsion triple, on procède comme dans l'exemple 2.

On obtient un gel translucide, onctueux.

## ANNEXE - MATIERES PREMIERES

| | |
|---|---|
| ABIL WE 09 R<br>Société GOLDSCHMIDT | : Cétyl diméthicone copolyol, polyglycéryl-4 isostéarate, hexyl laurate (33,3% / 33,3% / 33,4%) |
| VOLATIL SILICONE 7158 R<br>Société UNION CARBIDE | : Cyclopentadiméthylsiloxane |
| VISCASIL 60 M R<br>Société GENERAL ELECTRIC | : Polydiméthylsiloxane |
| GERMALL 115 R<br>Société ISP | : Imidazolidinylurée |
| PROPYLENEGLYCOL R<br>Société DOW CHEMICAL | : Propylèneglycol |
| GLYCERINE PUR CODEX R<br>Société STEARINERIE DUBOIS | : Glycérine |
| CARBOPOL 980 R<br>Société GOODRICH | : Polymère carboxyvinylique synthétisé dans le mélange acétate d'éthyle/cyclohexane |
| TRIETHANOLAMINE à 99% R<br>Société B.P. | : Triéthanolamine |
| SATIAGEL K 80 R<br>Société SANOFI | : Carraghénane pur (D-galactopyrannose sulfate) |
| KELTROL R<br>Société KELCO | : Xanthane/polysaccharides : glucose/mannose/acide glucuronique (40/30/30) |
| LUBRAJEL MS R<br>Société GUARDIAN | : Polyméthacrylate de glycéryle, glycérine, propylèneglycol, eau (3% / 47% / 1% / 48,8%) |

| | |
|---|---|
| PURCELLIN LIQUIDE HUILE 2/066210 - Société DRAGOGO | : Myristate d'isopropyle, : octanoate de cétéaryle (10/90) |
| CARBOPOL 1342 $^{R}$ Société GOODRICH | : Copolymère acide acrylique/ $C_{10}$-$C_{30}$ alkylacrylate (Dénomination CTFA : Acrylates/$C_{10}$-$C_{30}$ alkyl acrylate crosspolymer) |
| NIPASTAT $^{R}$ Société NIPA | : Méthylparaben, butylparaben, éthylparaben, propylparaben, isobutylparaben (57/15/14/7/7) |
| ABIL WAX 2434 $^{R}$ Société GOLDSCHMIDT | : Polystéaroxy diméthylsiloxane |
| DC QC F2 - 1671 $^{R}$ Société DOW CORNING | : Diméthiconol/cyclométhicone (12,5% / 87,5%) |
| CAFEINE $^{R}$ Société PROLABO | : Caféine |
| DC Q2 3225C $^{R}$ Société DOW CORNING | : Polydiméthysiloxane oxy-éthyléné, cyclométhicone (10/90) |
| DC FLUID 200 $^{R}$ Société DOW CORNING | : Polydiméthylsiloxane |
| DC FLUID 593 $^{R}$ Société DOW CORNING | : Triméthylsiloxysilicate/ polydiméthylsiloxane (33%/67%) |
| ABIL K03 $^{R}$ Société GOLDSCHMIDT | : Polydiméthylsiloxane |
| DC FLUID 345 $^{R}$ Société DOW CORNING | : Cyclopentadiméthylsiloxane/ cyclotétradiméthylsiloxane (75/25) |

| | | |
|---|---|---|
| MEXORYL SN R<br>Société CHIMEX | : | Thiolanediol = transthiolane diol-3,4 S - dioxyde |
| CB 185 R<br>Société RHONE POULENC | : | Copolymère de polydiméthyl-siloxane/benzylidènecamphre (silicone filtre) |
| ABIL AV 1000 R<br>Société GOLDSCHMIDT | : | Polyphényltriméthylsiloxane |
| HUILE CL 1680 R<br>Société RHONE POULENC | : | Monopentaérythritol triméthyl-silyle/cyclotétradiméthyl-siloxane (50/50) |
| PEMULEN TR 1 R<br>Société GOODRICH | : | Copolymère acide acrylique/ $C_{10}$-$C_{30}$ alkylacrylate (Dénomination CTFA : Acrylates/$C_{10}$-$C_{30}$ alkyl acrylate crosspolymer) |
| FL 100/450 R<br>Société SHIN ETSU | : | Trifluoroalkyldiméthylsiloxane |
| ABIL EM90 R<br>Société GOLDSCHMIDT | : | Cétyl diméthicone copolyol |
| UNITWIX<br>Société GUARDIAN | : | Glycoldistéarate + Tristéarin (diester d'éthylèneglycol et d'acide stéarique + triester de glycérol et d'acide stéarique) |

**Revendications**

1. Emulsion triple eau/huile de silicone/eau gélifiée, caractérisée par le fait qu'elle comporte :

   (A) une phase aqueuse externe continue gélifiée, comprenant au moins un gélifiant à chaîne grasse du type copolymère acide ou anhydride d'acide carboxylique monoéthylénique en $C_3$-$C_6$/ester acrylique à chaîne grasse;

(B) une phase grasse siliconée comprenant au moins une huile de silicone et un émulsionnant siliconé, et constituant avec une phase aqueuse, l'émulsion primaire E/H.

2. Emulsion triple selon la revendication 1, caractérisée par le fait que la phase aqueuse externe gélifiée comporte, en outre, un autre gélifiant choisi parmi les polymères carboxyvinyliques, les polyméthacrylates de glycéryle, les carraghénanes et les gommes de xanthane.

3. Emulsion triple selon les revendications 1 ou 2, caractérisée par le fait que la phase aqueuse externe gélifiée contient en outre des glycols, des neutralisants, des conservateurs, des colorants, des parfums, des substances actives, des filtres solaires, des hydratants et des agents d'onctuosité.

4. Emulsion triple selon l'une quelconque des revendications 1 à 3, caractérisée par le fait que l'émulsion primaire E/H contient au moins un composé comportant des chaînons organofluorés et des chaînons hydrocarbonés.

5. Emulsion triple selon la revendication 4, caractérisée par le fait que le composé comportant des chaînons organofluorés et des chaînons hydrocarbonés a un taux de substitution compris entre 0,5 et 95%.

6. Emulsion triple selon l'une quelconque des revendications 1 à 5, caractérisée par le fait que la phase grasse de l'émulsion primaire comprend, en outre, au moins une gomme de silicone, une cire de silicone et/ou une résine de silicone.

7. Emulsion triple selon l'une quelconque des revendications 1 à 6, caractérisée par le fait que la phase grasse de l'émulsion primaire comprend, en outre, des huiles non siliconées choisies parmi les huiles végétales ou animales, les huiles minérales ou synthétiques et les triglycérides d'acides gras, ainsi que des substances actives et des filtres solaires.

8. Emulsion triple selon l'une quelconque des revendications 1 à 7, caractérisée par le fait que la phase aqueuse de l'émulsion primaire comprend outre de l'eau, des sels stabilisant l'émulsion, des hydratants, des polyols, des substances actives, des conservateurs, des filtres solaires, des parfums et des colorants.

9. Emulsion triple selon l'une quelconque des revendications 1 à 8, caractérisée par le fait que la viscosité de l'émulsion primaire est comprise entre 0,1 Pa.s et 3 Pa.s, de préférence entre 0,13 Pa.s et 1,1 Pa.s.

10. Emulsion triple selon l'une quelconque des revendications 1 à 9, caractérisée par le fait que le rapport des proportions dans l'émulsion triple de l'émulsionnant siliconé de l'émulsion primaire au gélifiant à chaîne grasse de la phase aqueuse externe est compris entre 0,1 et 1,75, et de préférence entre 0,2 et 1,2.

11. Emulsion triple selon l'une quelconque des revendications 1 à 10, caractérisée par le fait qu'elle comprend, par rapport au poids total de l'émulsion triple, 0,05 à 8% d'un émulsionnant siliconé, 0,25 à 60% d'une huile de silicone, 0 à 16% d'une gomme de silicone, 0 à 16% d'une cire de silicone, 0 à 8% d'une résine de silicone, 0 à 25% en poids de composé à chaînons organofluorés et hydrocarbonés, 0,1 à 3% d'un gélifiant à chaîne grasse, 0,1 à 3% d'un neutralisant, 0 à 50% de glycol, des sels choisis parmi le chlorure de sodium, le chlorure de magnésium et le sulfate de magnésium, en quantité suffisante pour stabiliser l'émulsion primaire, ainsi qu'éventuellement des substances actives cosmétologiques ou dermatologiques, et tout autre adjuvant habituellement utilisé en cosmétique, le complément étant constitué par de l'eau.

12. Emulsion triple selon l'une quelconque des revendications 1 à 11, caractérisée par le fait que l'émulsionnant siliconé contenu dans l'émulsion primaire E/H est choisi parmi les diméthicone copolyols, et de préférence les alkyldiméthicone copolyols.

13. Emulsion triple selon l'une quelconque des revendications 1 à 12, caractérisée par le fait que l'huile de silicone contenue dans l'émulsion primaire E/H est choisie parmi les cyclométhicones, les polydiméthylsiloxanes (diméthicones), les polyphényltriméthylsiloxanes (phényltriméthicones), le monopentaérythritol triméthylsilyle et les silicones fluorées telles que les polydiméthylsiloxanes greffés par des groupes trifluoroalkyle.

14. Emulsion triple selon l'une quelconque des revendications 1 à 13, caractérisée par le fait que la gomme de silicone est choisie parmi les polydiméthylsiloxanes (diméthicones) de haut poids moléculaire et les polydiméthylsiloxane-ol (diméthicone-ol).

15. Emulsion triple selon l'une quelconque des revendications 1 à 14, caractérisée par le fait que la cire de silicone est choisie parmi les alcoxypolydiméthylsiloxanes (alcoxydiméthicones), les alkylpolysiloxanes (alkyldiméthicones) et les polydiméthylsiloxanes à fonction mercapto.

16. Emulsion triple selon l'une quelconque des revendications 1 à 15, caractérisée par le fait que la résine de silicone est choisie parmi les triméthylsiloxysilicates.

17. Procédé de préparation d'une émulsion triple selon l'une quelconque des revendications précédentes, caractérisé par le fait que, dans un premier temps, on réalise l'émulsion primaire E/H en ajoutant une phase aqueuse à une phase grasse siliconée et, dans un deuxième temps, on réalise l'émulsion triple par addition de l'émulsion primaire obtenue précédemment à une seconde phase aqueuse externe continue gélifiée, l'émulsion primaire contenant au maximum 10% en poids d'huile de silicone, par rapport au poids total de l'émulsion triple finale.

18. Procédé de préparation d'une émulsion triple selon l'une quelconque des revendications 1 à 16, caractérisé par le fait que, dans un premier temps, on réalise l'émulsion primaire E/H en ajoutant une phase aqueuse à une phase grasse siliconée comprenant au moins un émulsionnant siliconé et une huile siliconée ou non siliconée, dans un deuxième temps, on dilue l'émulsion primaire obtenue avec une huile de silicone identique à ou différente de celle(s) éventuellement utilisée(s) dans l'émulsion primaire et enfin, dans un troisième temps, on réalise l'émulsion triple par addition de l'émulsion primaire diluée à une seconde phase aqueuse externe continue gélifiée.

19. Composition cosmétique destinée à être appliquée sur la peau ou les cheveux, caractérisée par le fait qu'elle comporte au moins une émulsion triple telle que définie dans l'une quelconque des revendications 1 à 16.

20. Composition destinée à être utilisée en dermatologie et contenant des agents dermatologiquement actifs, caractérisée par le fait que son support est constitué par une émulsion triple telle que définie dans l'une quelconque des revendications 1 à 16.

**Claims**

1. Gelled triple water/silicone oil/water emulsion, characterized in that it comprises:

   (A) a gelled, continuous, external aqueous phase which comprises at least one fatty-chain gelling agent of the $C_3$-$C_6$ monoethylenic carboxylic acid or anhydride/fatty-chain acrylic ester copolymer type;
   (B) a silicone-containing fatty phase comprising at least one silicone oil and a silicone-containing emulsifier, forming the primary W/O emulsion with an aqueous phase.

2. Triple emulsion according to claim 1, characterized in that the gelled external aqueous phase additionally comprises another gelling agent chosen from carboxyvinyl polymers, glyceryl polymethacrylates, carragheenans and xanthan gums.

3. Triple emulsion according to claims 1 or 2, characterized in that the gelled external aqueous phase additionally contains glycols, neutralizing agents, preservatives, dyes, fragrances, active substances, sunscreens, moisturizers and unctuosity agents.

4. Triple emulsion according to any one of claims 1 to 3, characterized in that the primary W/O emulsion contains at least one compound comprising organofluorine chain members and hydrocarbon chain members.

5. Triple emulsion according to claim 4, characterized in that the compound comprising organofluorine chain members and hydrocarbon chain members has a degree of substitution of between 0.5 and 95%.

6. Triple emulsion according to any one of claims 1 to 5, characterized in that the fatty phase of the primary emulsion additionally comprises at least one silicone gum, one silicone wax and/or one silicone resin.

7. Triple emulsion according to any one of claims 1 to 6, characterized in that the fatty phase of the primary emulsion additionally comprises non-silicone oils chosen from vegetable or animal oils, mineral or synthetic oils and fatty acid triglycerides, and also active substances and sunscreens.

8. Triple emulsion according to any one of claims 1 to 7, characterized in that the aqueous phase of the primary emulsion comprises, besides water, salts which stabilize the emulsion, moisturizers, polyols, active substances, preservatives, sunscreens, fragrances and dyes.

9. Triple emulsion according to any one of claims 1 to 8, characterized in that the viscosity of the primary emulsion is between 0.1 Pa.s and 3 Pa.s, preferably between 0.13 Pa.s and 1.1 Pa.s.

10. Triple emulsion according to any one of claims 1 to 9, characterized in that the ratio of the proportions, in the triple emulsion, of the silicone-containing emulsifier of the primary emulsion to the fatty-chain gelling agent of the external aqueous phase is between 0.1 and 1.75 and preferably between 0.2 and 1.2.

11. Triple emulsion according to any one of claims 1 to 10, characterized in that it comprises, relative to the total weight of the triple emulsion, from 0.05 to 8% of a silicone-containing emulsifier, from 0.25 to 60% of a silicone oil, from 0 to 16% of a silicone gum, from 0 to 16% of a silicone wax, from 0 to 8% of a silicone resin, from 0 to 25% by weight of compound containing organofluorine and hydrocarbon chain members, from 0.1 to 3% of a fatty-chain gelling agent, from 0.1 to 3% of a neutralizing agent, and from 0 to 50% by weight of glycol, and of salts chosen from sodium chloride, magnesium chloride and magnesium sulphate, in a quantity which is sufficient to stabilize the primary emulsion, and also, optionally, cosmetological or dermatological active substances, and any other adjuvant which is conventionally used in cosmetics, the remainder consisting of water.

12. Triple emulsion according to any one of claims 1 to 11, characterized in that the silicone-containing emulsifier present in the primary W/O emulsion is chosen from dimethicone copolyols and preferably alkyl dimethicone copolyols.

13. Triple emulsion according to any one of claims 1 to 12, characterized in that the silicone oil present in the primary W/O emulsion is chosen from cyclomethicones, polydimethylsiloxanes (dimethicones), polyphenyltrimethylsiloxanes (phenyl trimethicones), monopentaerythritol trimethylsilyl and fluorinated silicones such as polydimethylsiloxanes grafted with trifluoroalkyl groups.

14. Triple emulsion according to any one of claims 1 to 13, characterized in that the silicone gum is chosen from polydimethylsiloxanes (dimethicones) of high molecular weight and polydimethylsiloxane-ols (dimethicone-ols).

15. Triple emulsion according to any one of claims 1 to 14, characterized in that the silicone wax is chosen from alkoxypolydimethylsiloxanes (alkoxy dimethicones), alkylpolysiloxanes (alkyl dimethicones) and polydimethylsiloxanes having a mercapto functional group.

16. Triple emulsion according to any one of claims 1 to 15, characterized in that the silicone resin is chosen from trimethylsiloxysilicates.

17. Process for the preparation of a triple emulsion according to any one of the preceding claims, characterized in that, in a first stage, the primary W/O emulsion is prepared by adding an aqueous phase to a silicone-containing fatty phase and, in a second stage, the triple emulsion is prepared by addition of the primary emulsion obtained beforehand to a second, gelled, continuous, external aqueous phase, the primary emulsion containing not more than 10% by weight of silicone oil relative to the total weight of the final triple emulsion.

18. Process for the preparation of a triple emulsion according to any one of claims 1 to 16, characterized in that, in a first stage, the primary W/O emulsion is prepared by adding an aqueous phase to a silicone-containing fatty phase comprising at least one silicone-containing emulsifier and a silicone or non-silicone oil, in a second stage the primary emulsion obtained is diluted with a silicone oil which is identical to or different from that (those) optionally used in the primary emulsion, and finally, in a third stage, the triple emulsion is prepared by addition of the diluted primary emulsion to a second, gelled, continuous, external aqueous phase.

19. Cosmetic composition intended for application to the skin or hair, characterized in that it comprises at least one triple emulsion as defined in any one of claims 1 to 16.

20. Composition intended for use in dermatology and containing dermatologically active agents, characterized in that its vehicle consists of a triple emulsion as defined in any one of claims 1 to 16.

## Patentansprüche

1. Wasser/Öl-Dreifach-Emulsion aus Silikongeliertem Wasser, dadurch **gekennzeichnet,** daß sie umfaßt:

   (A) eine wässrige äußere kontinuierliche gelierte Phase, die mindestens ein Geliermittel mit Fettkette vom Typ eines Copolymer aus einer monoethylenischen $C_{3-6}$-Carboxylsäure oder einem entsprechenden Säureanhydrid und aus einem Acrylester mit Fettkette enthält,

   (B) eine silikonhaltige Fettphase, die mindestens ein Silikon-Öl und einen silikonhaltigen Emulgator enthäl, und mit einer wässrigen Phase eine W/O-Primäremulsion aufbaut.

2. Dreifach-Emulsion gemäß Anspruch 1, dadurch **gekennzeichnet,** daß die wässrige äußere gelierte Phase, zusätzlich, ein weiteres Geliermistel enthält, das aus Carboxyvinyl-Polymeren, Glycerylpolymethacrylaten, Karraghenanen und aus Gummiprodukten von Xanthan ausgewählt ist.

3. Dreifach-Emulsion gemäß der Ansprüche 1 oder 2, dadurch **gekennzeichnet,** daß die wässrige äußere gelierte Phase ausserdem Glycole, Neutralisiermittel, Konservierungsmittel, Färbemittel, Parfüm-Produkte, Aktivsubstanzen, Sonnen-Filterstoffe, Hydratisiermittel und salbende Mittel enthält.

4. Dreifach-Emulsion gemäß jedem der Ansprüche 1 bis 3, dadurch **gekennzeichnet,** daß die W/O-Primäremulsion mindestens eine Verbindung enthält, die organofluorierte Ketten und Kohlenwasserstoffketten aufweist.

5. Dreifach-Emulsion gemäß Anspruch 4, dadurch **gekennzeichnet,** daß die Verbindung mit organofluorierten Ketten und Kohlenwasserstoffketten einen Substitutionsgrad von 0,5 bis 95% aufweist.

6. Dreifach-Emulsion gemäß jedem der Ansprüche 1 bis 5, dadurch **gekennzeichnet,** daß die Fettphase der Primäremulsion, zusätzlich, mindestens einen Silikon-Gummi, ein Silikon-Wachs und/oder ein Silikon-Harz enthält.

7. Dreifach-Emulsion gemäß jedem der Ansprüche 1 bis 6, dadurch **gekennzeichnet,** daß die Fettphase der Primäremulsion, zusätzlich, nichtsilikonhaltige Öle, ausgewählt aus pflanzlichen oder tierischen Ölen, mineralischen oder synthetischen Ölen und aus Triglyceriden von Fettsäuren, sowie Wirksubstanzen und Sonnen-Filterstoffe enthält.

8. Dreifach-Emulsion gemäß jedem der Ansprüche 1 bis 7, dadurch **gekennzeichnet,** daß die wässrige Phase der Primäremulsion ausser Wasser Salze zur Stabilisierung der Emulsion, Hydratisiermittel, Polyole, Wirksubstanzen, Konservierungsstoffe, Sonnen-Filterstoffe, Parfüm-Produkte und Färbemittel enthält.

9. Dreifach-Emulsion gemäß jedem der Ansprüche 1 bis 8, dadurch **gekennzeichnet,** daß die Viskosität der Primäremulsion 0,1 bis 3, vorzugsweise 0,13 bis 1,1, Pa x s beträgt.

10. Dreifach-Emulsion gemäß jedem der Ansprüche 1 bis 9, dadurch **gekennzeichnet,** daß das Verhältnis der Anteilsmengen in der Dreifach-Emulsion des silikonhaltigen Emulgators der Primäremulsion zum Geliermittel mit Fettkette der wässrigen äußeren Phase 0,1 bis 1,75, vorzugsweise 0,2 bis 1,2, beträgt.

11. Dreifach-Emulsion gemäß jedem der Ansprüche 1 bis 10, dadurch **gekennzeichnet,** daß

sie, bezogen auf das Gesamtgewicht der Dreifach-Emulsion, 0,05 bis 8% silikonhaltigen Emulgator, 0,25 bis 60% Silikon-Öl, 0 bis 16% Silikon-Gummi, 0 bis 16% Silikon-Wachs, 0 bis 8% Silikon-Harz, 0 bis 25% Verbindung mit organofluorierten und Kohlenwasserstoffketten, 0,1 bis 3% Geliermittel mit Fettkette, 0,1 bis 3% Neutralisiermittel, 0 bis 50% Glycol, Salze, ausgewählt aus Natriumchlorid, Magnesiumchlorid und Magnesiumsulfat, in einer zur Stabilisierung der Primäremulsion ausreichenden Menge sowie gegebenenfalls kosmetologische oder dermatologische Wirksubstanzen und jeden weiteren gewöhnlich in der Kosmetik verwendeten Hilfsstoff enthält, wobei die ergänzende Restmenge aus Wasser Zusammengesetzt ist.

**12.** Dreifach-Emulsion gemäß jedem der Ansprüche 1 bis 11,
dadurch **gekennzeichnet,** daß
der in der W/O-Primäremulsion enthaltene silikonhaltige Emulgator aus Dimethicon-Copolyolen und vorzugsweise aus Alkyldimethicon-Copolyolen ausgewählt ist.

**13.** Dreifach-Emulsion gemäß jedem der Ansprüche 1 bis 12,
dadurch **gekennzeichnet,** daß
das in der W/O-Primäremulsion enthaltene Silikon-Öl aus Cyclomethiconen, Polydimethylsiloxanen (Dimethiconen), Polyphenyltrimethylsiloxanen (Phenyltrimethiconen), Silan-Trimethylsilylmonopentaerythrit und aus fluorierten Silikonen wie aus mit Trifluoralkylgruppen gepfropften Polydimethylsiloxanen ausgwählt ist.

**14.** Dreifach-Emulsion gemäß jedem der Ansprüche 1 bis 13,
dadurch **gekennzeichnet,** daß
der Silikon-Gummi aus Polydimethylsiloxanen (Dimethiconen) hohen Molekulargewichts und aus Polydimethylsiloxan-ol (Dimethicon-ol) ausgewählt ist.

**15.** Dreifach-Emulsion gemäß jedem der Ansprüche 1 bis 14,
dadurch **gekennzeichnet,** daß
das Silikon-Wachs aus Alkoxypolydimethylsiloxanen (Alkoxyidimethiconen), Alkylpolysiloxanen (Alkyldimethiconen) und aus Polydimethylsiloxanen mit Mercapto-Funktion ausgewählt ist.

**16.** Dreifach-Emulsion gemäß jedem der Ansprüche 1 bis 15,
dadurch **gekennzeichnet,** daß
das Silikon-Harz aus Trimethylsiloxysilikaten ausgewählt ist.

**17.** Verfahren zur Herstellung einer Dreifach-Emulsion gemäß jedem der vorhergehenden Ansprüche,
dadurch **gekennzeichnet,** daß
man, in einer ersten Stufe, die W/O-Primäremulsion herstellt, wobei man eine wässrige Phase einer silikonhaltigen Fettphase zufügt, und daß man, in einer zweiten Stufe, die Dreifach-Emulsion herstellt, indem man die vorher erhaltene Primäremulsion einer wässrigen äußeren kontinuierlichen gelierten zweiten Phase zufügt, wobei die Primäremulsion maximal 10 Gew.% Silikon-Öl enthält, bezogen auf das Gesamtgewicht der Dreifach-Emulsion.

**18.** Verfahren zur Herstellung einer Dreifach-Emulsion gemäß jedem der Ansprüche 1 bis 16,
dadurch **gekennzeichnet,** daß
man, in einer ersten Stufe, die W/O-Primäremulsion herstellt, indem man eine wässrige Phase einer silikonhaltigen Fettphase zufügt, die mindestens einen silikonhaltigen Emulgator und ein silikonhaltiges oder nicht-silikonhaltiges Öl enthält, daß man in einer zweiten Stufe die erhaltene Primäremulsion mit einem Silikon-Öl verdünnt, das gleich oder verschieden mit bzw. zu den gegebenenfalls in der Primäremulsion eingesetzten ist, und daß man schließlich, in einer dritten Stufe, die Dreifach-Emulsion herstellt, indem man die verdünnte Primäremulsion zu einer wässrigen äußeren kontinuierlichen gelierten zweiten Phase gibt.

**19.** Kosmetische Zusammensetzung zur Aufbringung auf die Haut oder die Haare,
dadurch **gekennzeichnet,** daß
sie mindestens eine in jedem der Anprüche 1 bis 16 definierte Dreifach-Emulsion enthält.

**20.** Zusammensetzung zur Anwendung in der Dermatologie, welche dermatologisch wirksame Mittel enthält,
dadurch **gekennzeichnet,** daß
ihre Trägergrundlage aus einer in jedem der Ansprüche 1 bis 16 definierten Dreifach-Emulsion zusammengesetzt ist.